# EUROPÄISCHE PATENTSCHRIFT

(11) **EP 1 175 592 B1**
(45) Veröffentlichungstag und Bekanntmachung des Hinweises auf die Patenterteilung: **11.06.2003**
(21) Anmeldenummer: 99916740.6
(22) Anmeldetag: 03.05.1999
(51) Int. Cl.: G01B 11/16, A61B 6/00

(54) **POSITIONSERFASSUNGSVORRICHTUNG MIT HILFSMITTELN ZUR ERMITTLUNG DER RICHTUNG DES SCHWERKRAFTVEKTORS**
POSITION DETECTOR WITH AUXILIARY MEANS FOR DETECTING THE DIRECTION OF THE GRAVITY VECTOR
DISPOSITIF DE DETECTION DE POSITION DOTE DE MOYENS AUXILIAIRES PERMETTANT DE DETERMINER LA DIRECTION DU VECTEUR DE GRAVITE

(43) Veröffentlichungstag der Anmeldung: 30.01.2002
(73) Patentinhaber: SYNTHES AG Chur, 7002 Chur (CH)
(72) Erfinder: HOFSTETTER, Robert, CH-3007 Bern (CH); GUGGENHEIM, Nicolas, CH-4147 Aesch (CH); SCHERRER, José, L., CH-4702 Oensingen (CH)
(74) Vertreter: Lusuardi, Werther Giovanni, Dr.
(86) Internationale Anmeldenummer: CH9900183
(87) Internationale Veröffentlichungsnummer: WO00066971

(56) Entgegenhaltungen:
- DE-A- 19 711 964
- FR-A- 2 608 271
- US-A- 4 652 917

## Beschreibung

Die Erfindung bezieht sich auf eine Vorrichtung zur Erfassung der Position und Orientierung von mit je mindestens drei nicht-kollinear angeordneten Markern versehenen Körpern innerhalb mindestens eines dreidimensionalen Koordinatensystems gemäss dem Oberbegriff des Patentanspruchs 1 und auf ein Verfahren zur Korrektur von Röntgenbildern gemäss dem Oberbegriff des Patentanspruchs 24.

Der Einsatz von im Raum bewegbaren Messgeräten oder bildgebenden Geräten erfordert vielfach je nach Messung eine Veränderung der Position oder Orientierung des Messgerätes. Dies gilt auch für Röntgengeräte, insbesondere auch für die häufig im Operationsraum verwendeten mobilen und/oder schwenkbaren Röntgengeräte (Bildverstärker, C-Arm Geräte).

Ein System zur Erfassung der Position und Orientierung eines chirurgischen Instrumentes oder Gerätes innerhalb eines Objektes und zur gleichzeitigen Anzeige von vorher aufgenommenen, zur erfassten Position entsprechenden Bildern des Objektes ist aus der Patentschrift US 5,383,454 BUCHHOLZ bekannt. Dieses System ermöglicht ebenfalls, die Spitze eines Messfühlers in einem Objekt an einen bestimmten Ort hinzuführen, wobei die Lage des Messfühlers nur an einem Bildschirm, welcher gleichzeitig auch ein vorher aufgenommenes Bild dieses Teils des Objektes wiedergibt, beobachtet werden kann. Die Position des Messfühlers wird bei dieser bekannten Erfindung durch einen handelsüblichen dreidimensionalen Schalldigitizer ermittelt.

Die Geräteorientierung kann bei schweren Geräten, wie im Operationsraum eingesetzten Röntgengeräten, je nach Ausrichtung des Gerätes zum Gravitationsfeld der Erde aufgrund von Materialdeformationen die Messung, respektive Bilderfassung beeinflussen. Ebenfalls können bei Röntgengeräten mit magneto-optischer Bilderfassung die Röntgenbilder je nach Ausrichtung des Gerätes zum Erdmagnetfeld beeinträchtigt werden. Eine weitere mögliche Deformation dieser Röntgenbildern beruht auf dem Einfluss von optischen Deformationen im Empfänger, welche insbesondere von der Beschaffenheit der Strahlenquelle und des Empfänger abhängig sind und beispielsweise durch die Umwandlung von Elektronen in Photonen und eine weitere Umwandlung der Photonen in eine elektrisches Signal hervorgerufen werden können.

Hier will die Erfindung Abhilfe schaffen. Der Erfindung liegt die Aufgabe zugrunde, zusätzlich den lokalen Schwerkraftvektor in-situ zu messen und die daraus folgenden Korrekturen hinsichtlich des Einflusses von schwerkraftbedingten Deformationen des Röntgengerätes, welche bei horizontaler beziehungsweise vertikaler Ausrichtung des Röntgengerätes verschieden sind, sowie der Einflüsse der Schwerkraft und des Erdmagnetfeldes auf die Messwerte oder Röntgenbilder zu ermitteln und diese entsprechend zu korrigieren.

Die Erfindung löst die gestellte Aufgabe mit einer Vorrichtung zur Erfassung der Position und Orientierung von mit je mindestens drei nicht-kollinearen Markern versehenen Körpern innerhalb mindestens eines dreidimensionalen Koordinatensystems, welche die Merkmale des Anspruchs 1 aufweist, sowie mit einem Verfahren zur Korrektur von Röntgenbildern, welches die Merkmale des Anspruchs 24 aufweist.

Weitere vorteilhafte Ausgestaltungen der Erfindung sind in den abhängigen Ansprüchen gekennzeichnet.

Die erfindungsgemässe Vorrichtung zur Positionserfassung mit Hilfsmitteln zur Erfassung und Referenzierung des Schwerkraftvektors umfasst im wesentlichen einen die Position von Markern, vorzugsweise Infrared Light Emitting Dioden (IRED), innerhalb eines Koordinatensystems erfassenden optoelektronischen Sensor, welcher mit einem Computer zur Auswertung der vom Sensor erhaltenen Signale verbunden ist, und Hilfsmittel, welche die Richtung des Schwerkraftvektors bezüglich desselben dreidimensionalen Koordinatensystems erfassen und mittels des Computers eine Koordinatentransformation zwischen einem sensorfesten Koordinatensystem und einem raumfesten oder schwerkraftfesten Koordinatensystem ermöglichen.

Anstelle der optoelektronischen Positionserfassung kann die erfindungsgemässe Vorrichtung auch eine akustische oder magnetische Positionserfassungseinheit umfassen.

Abhängig von der gewählten Positionserfassungseinheit können die Marker Sender, Empfänger oder Reflektoren sein. Beispielsweise sind als Sender:
- Lichtquellen;
- Light emitting dioden (LED's);
- Infrared light emitting dioden (IRED's);
- Akustische Sender;
- Spulen zum Aufbau eines Magnetfeldes; oder
- Reflektoren mit entsprechenden Sendern in einer örtlich getrennten Einheit;
oder als Empfänger:
- CCD-Kameras;
- Photodioden;
- Mikrophone; oder
- Hall-effekt Komponenten;
denkbar.

In einer bevorzugten Ausführungsform der erfindungsgemässen Vorrichtung umfasst diese einen Positionserfassungssensor mit mindestens zwei optoelektronischen Kameras, welche vorzugsweise mit CCD-Chips (Charge-coupled device chips) ausgestattet sind, ein erstes Inklinometer, dessen Horizontalachse die x-Achse eines schwerkraftfesten dreidimensionalen Koordinatensystems definiert, ein zweites Inklinometer, dessen Horizontalachse die y-Achse des schwerkraftfesten dreidimensionalen Koordinatensystems definiert, und einen Computer, welcher mit Software zur dreidimensionalen realtime Betrachtung der Daten am Bildschirm in numerischer oder graphischer Form ausgerüstet ist. Durch die Horizontalachsen der Inklinometer wird eine senkrecht zum Schwerkraftvektor stehende Ebene aufgespannt, welche zusammen mit dem Schwerkraftvektor ein dreidimensionales schwerkraftfestes Koordinatensystem bilden. Durch elektronische Übertragung der von den Inklinometern abgegebenen Signale zum Computer lässt sich eine Abweichung der Parallelität zwischen einer der sensorfesten Koordinatenachsen von der entsprechenden schwerkraftfesten Koordinatenachse feststellen und die Drehung oder Drehungen des sensorfesten Koordinatensystems gegenüber dem schwerkraftfesten Koordinatensystems ermitteln.

Die senkrecht zum Schwerkraftvektor stehende Ebene lässt sich bei geeigneten Bedingungen auch mit nur einem Inklinometer ermitteln.

Als Inklinometer sind Geräte einsetzbar, welche auf dem Gasblasen/Flüssigkeits-Prinzip (Wasserwaage), Kreiselprinzip (analog zum Kreiselkompass) oder Trägheitsprinzip (analog zur Trägheitspositionsbestimmung) arbeiten und Abweichungen gegenüber dem Schwerkraftvektor elektronisch erfassen. Geräte, welche nach dem Kreiselprinzip oder dem Trägheitsprinzip arbeiten, müssen vorgängig bezüglich ihrer Ausrichtung zum Schwerkraftvektor kalibriert werden. Ebenfalls möglich sind magnetische Geräte (analog zum elektronischen Kompass).

Als Positionserfassungsvorrichtung kann ein auf dem Markt erhältliches System, beispielsweise ein OPTOTRAK 3020 System, Northern Digital, Waterloo, On. eingesetzt werden.

Eine weitere Ausführungsform der erfindungsgemässen Vorrichtung unterscheidet sich von der oben beschriebenen Ausführungsform nur darin, dass die Ermittlung des Schwerkraftvektors durch einen sich im Schwerefeld ausrichtenden Körper erfolgt. Der Körper ist an einem dünnen Faden oder Draht aufgehängt und mit mindestens zwei in einem Abstand A angeordneten Markern ausgestattet. Die Position der Marker wird durch die Positionserfassungsvorrichtung detektiert, wodurch sich die Positionen der Marker im Raum und damit die Richtung des Schwerkraftvektors mittels des Computers ermitteln lassen. Die Richtung des Schwerkraftvektors im sensorfesten Koordinatensystem wird durch die Positionserfassungsvorrichtung erfasst und vom Computer mittels digitaler Datenverarbeitung die Drehung oder Drehungen des sensorfesten Koordinatensystems gegenüber dem schwerkraftfesten Koordinatensystem ermittelt.

Anstelle der Aufhängung des sich im Schwerefeld ausrichtenden Körpers an einem Faden oder Draht ist auch eine Aufhängung an einer Kette, eine kardanische Aufhängung, eine Lagerung mit einem Kugelgelenk, eine Einlagerung des Körpers in ein Elastomer, beispielsweise Silikonkautschuk oder geschäumter Silikonkautschuk möglich.

Vorzugsweise wird die Ausrichtung des Körpers im Schwerefeld gedämpft, was sich mechanisch, elektromagnetisch, durch ein den Körper umgebendes Flüssigkeitsbad, Federn oder Luft- oder Gasdruckstossdämpfer erreichen lässt.

Das erfindungsgemässe Verfahren zur Erfassung der genauen Ausrichtung eines mit Markern versehenen Röntgengerätes mit einer Röntgenquelle und einem ein Röntgenbild, vorzugsweise ein magneto-optisches Röntgenbild erzeugenden Empfänger im Schwerkraftfeld umfasst folgende Schritte:
a) Erfassen und Speichern der Richtung des Schwerkraftvektors innerhalb eines dreidimensionalen Koordinatensystems mittels einer Ausführungsform der erfindungsgemässen Vorrichtung, wobei das Koordinatensystem sensorfest, raumfest oder schwerkraftfest sein kann;
b) Ermitteln der Position der Röntgenquelle und des Empfängers innerhalb desselben Koordinatensystems mittels einer Ausführungsform der erfindungsgemässen Vorrichtung;
c) Ermitteln der aufgrund von schwerkraftbedingten mechanischen Deformationen des Röntgengerätes auftretenden Verzerrungen des Röntgenbildes mittels des Computers; und
d) Korrektur des Röntgenbildes bezüglich der unter Schritt c) ermittelten Verzerrungen mittels des Computers.

In einer bevorzugten Anwendungsform der erfindungsgemässen Verfahren umfasst dieses auch die weiteren Schritte:
e) Ermitteln der Richtung der senkrecht auf dem Röntgenbild stehenden Bildnormalen innerhalb desselben Koordinatensystems aus der unter Schritt b) ermittelten Position und Orientierung des Empfängers oder der Röntgenquelle mittels des Computers;
f) Ermitteln der aufgrund der Abweichung der Bildnormalen von der Richtung des lokalen Erdmagnetfeldes auftretenden Defekte des auf magneto-optischer Basis vom Empfänger erzeugten Röntgenbildes mittels des Computers; und
g) Korrektur des Röntgenbildes bezüglich der unter Schritt f) ermittelten Defekte mittels des Computers.

In einer weiteren Anwendungsform der erfindungsgemässen Verfahren umfasst dieses auch die weiteren Schritte:
h) Ermitteln der Defekte auf das magneto-optische Röntgenbild, welche aufgrund von optischen Deformationen im Empfänger verursacht werden;
i) Korrektur des Röntgenbildes bezüglich der unter Schritt h) ermittelten Defekte mittels des Computers.

Die durch die Erfindung erreichten Vorteile sind im wesentlichen darin zu sehen, dass dank der erfindungsgemässen Vorrichtung der lokale Schwerkraftvektor in-situ erfassbar ist und mittels des erfindungsgemässen Verfahrens Korrekturen ermittelbar sind, welche die aufgrund von schwerkraftbedingten mechanischen Deformationen des Röntgengerätes auftretenden Verzerrungen des Röntgenbildes berücksichtigen. Dies gilt insbesondere bei schwenkbaren Röntgengeräten wo die mechanischen Deformationen bei horizontaler beziehungsweise vertikaler Ausrichtung des Röntgengerätes verschieden sind. Ebenfalls lassen sich die Einflüsse von Schwerkraft und Erdmagnetfeld auf die Röntgenbilder ermitteln und entsprechend korrigieren. Ein weiterer Vorteil der Erfindung liegt darin, dass kein Eichkörper im Strahlengang angebracht werden muss, so dass die Operation durch einen solchen nicht beeinträchtigt wird.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine schematische Darstellung einer bevorzugten Ausführungsform der erfindungsgemässen Vorrichtung;
Fig. 2 eine schematische Darstellung einer weiteren bevorzugten Ausführungsform der erfindungsgemässen Vorrichtung; und
Fig. 3 eine Illustration zu einer bevorzugten Ausführungsart des erfindungsgemässen Verfahrens.

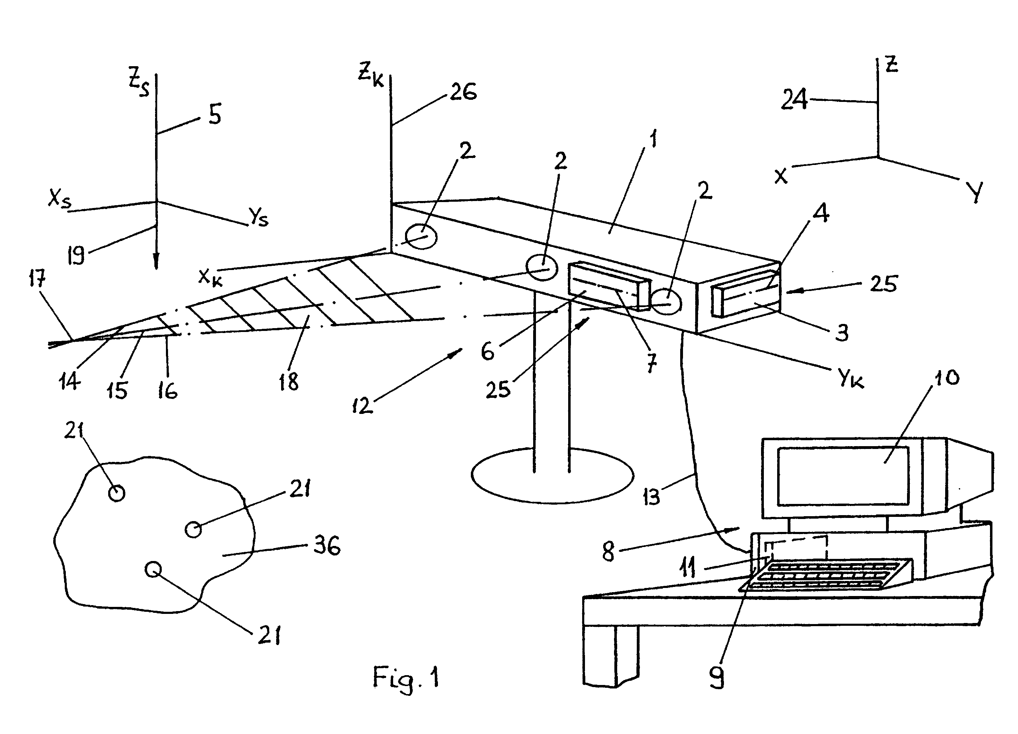

In Fig. 1 ist eine bevorzugte Ausführungsform der erfindungsgemässen Vorrichtung dargestellt. Die Vorrichtung umfasst einen Positionserfassungssensor 1 mit drei optoelektronischen Kameras 2, welche vorzugsweise mit linearen CCD-Chips (charge-coupled device chips) ausgestattet sind, ein mit dem Positionserfassungssensor 1 verbundenes erstes Inklinometer 3, dessen Horizontalachse 4 die x-Achse eines schwerkraftfesten dreidimensionalen Koordinatensystems 5 definiert, ein zweites mit dem Positionserfassungssensor 1 verbundenes Inklinometer 6, dessen Horizontalachse 7 rechtwinklig zur Horizontalachse 4 steht und die y-Achse des schwerkraftfesten dreidimensionalen Koordinatensystems 5 definiert, und einen Computer 8. Zur Verarbeitung der durch die Kameras 2 detektierten Signale ist die Positionserfassungsvorrichtung 12 (beispielsweise OPTOTRAK 3020, Northern Digital, Waterloo, Ont.) mit einer mit dem Computer 8 verbundenen System-Kontrolleinheit 9, Kabeln 13 und einer Interface-Karte 11 sowie mit Software zur dreidimensionalen realtime Betrachtung der Daten am Bildschirm 10 in numerischer oder graphischer Form ausgestattet. Die optischen Achsen 14;15;16 der Kameras 2 schneiden sich in einem Punkt 17 und bilden eine Ebene 18, welche dann senkrecht zum Schwerkraftvektor 19 steht, wenn die Koordinatenachse x_{K} des sensorfesten dreidimensionalen Koordinatensystems 26 parallel zur Horizontalachse 4 des ersten Inklinometers 3 und die Koordinatenachse y_{K} des sensorfesten Koordinatensystems 26 parallel zur Horizontalachse 7 des zweiten Inklinometers 6 ausgerichtet sind. Bei einer Abweichung der Parallelität zwischen einer der sensorfesten Koordinatenachsen x_{K};y_{K} von der entsprechend zugeordneten Horizontalachse 4;7 der Inklinometer 3;6 wird die Abweichung durch die Inklinometer 3;6 erfasst und vom Computer 8 mittels digitaler Signalverarbeitung der von den Inklinometern 3;6 erfassten Abweichungen die Drehung oder Drehungen des sensorfesten Koordinatensystems 26 gegenüber dem schwerkraftfesten Koordinatensystems 5 ermittelt.

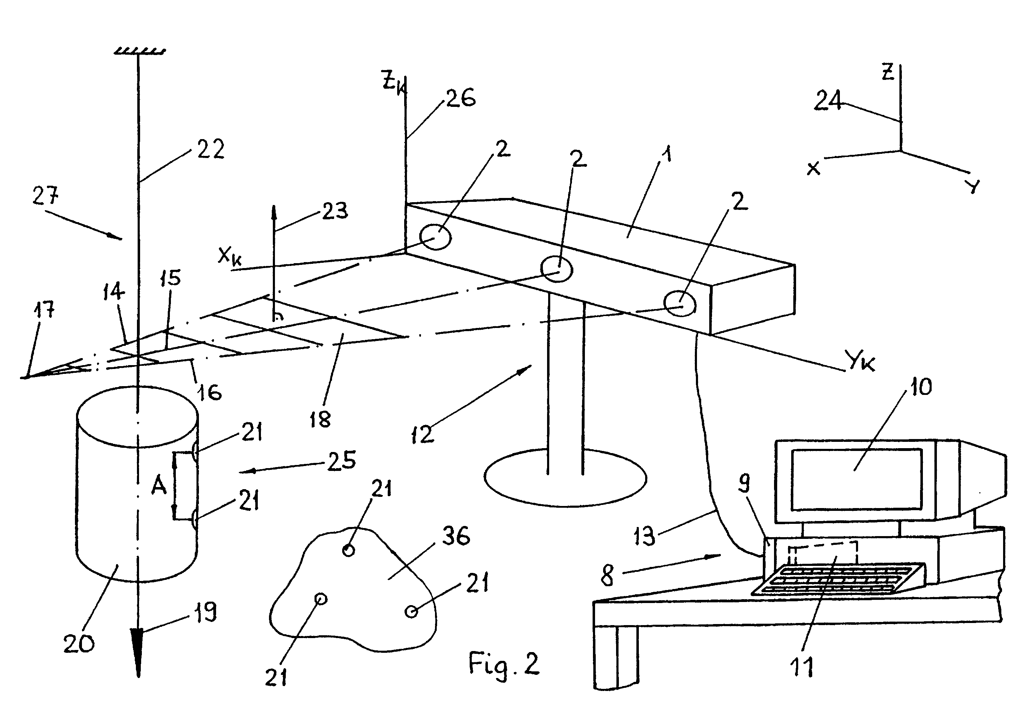

In Fig. 2 ist eine weitere bevorzugte Ausführungsform der erfindungsgemässen Vorrichtung dargestellt, welche sich von der in Fig. 1 dargestellten Ausführungsform nur darin unterscheidet, dass die Ermittlung des Schwerkraftvektors 19 durch einen sich im Schwerefeld ausrichtenden Körper 20 erfolgt. Der Körper 20 ist an einem dünnen Faden oder Draht 22 aufgehängt und mit mindestens zwei in einem Abstand A angeordneten Markern 21, vorzugsweise Infrared Light Emitting Dioden (IRED) ausgestattet. Das von den Markern 21 abgestrahlte Infrarotlicht wird durch die Kameras 2 der Positionserfassungsvorrichtung 12 detektiert, wodurch sich die Positionen der Marker 21 im Raum und damit die Richtung des Schwerkraftvektors 19 mittels des Computers 8 ermitteln lassen. Bei einer Abweichung der Parallelität zwischen der sensorfesten, senkrecht auf der Ebene 18 der optischen Achsen 14;15;16 der Kameras 2 stehenden Flächennormalen 23 und dem Schwerkraftvektor 19 wird die Abweichung durch den Positionserfassungssensor 1 erfasst und vom Computer 8 mittels digitaler Signalverarbeitung der vom Positionserfassungssensor 1 erfassten Abweichung die Drehung oder Drehungen des sensorfesten Koordinatensystems 26 gegenüber dem schwerkraftfesten Koordinatensystems 5 ermittelt.

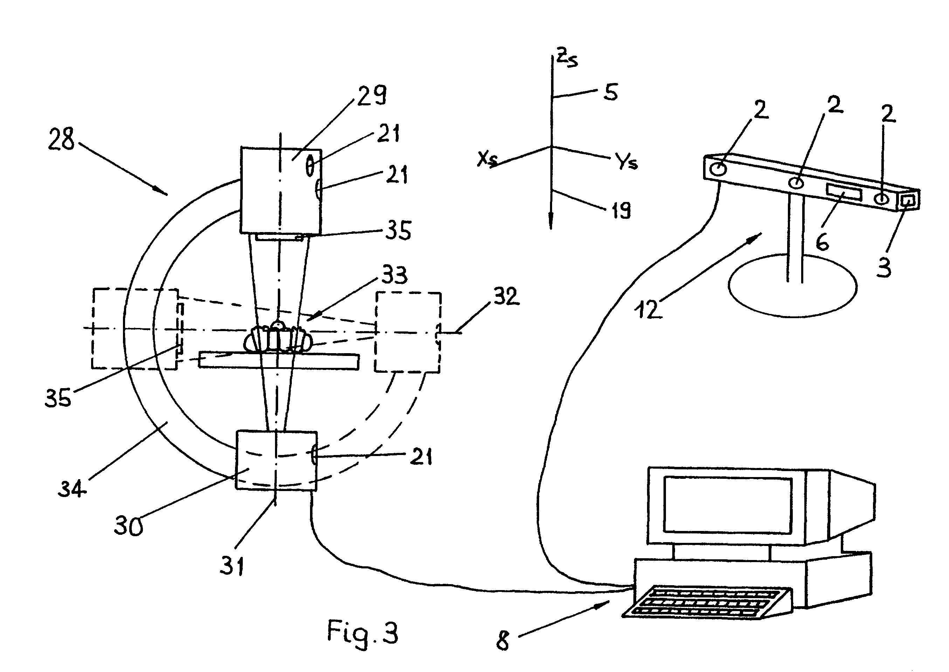

In Fig. 3 ist das erfindungsgemässe Verfahren zur Erfassung der genauen Ausrichtung eines Röntgengerätes 28 mit Hilfe der erfindungsgemässen Vorrichtung illustriert. Das Röntgengerät 28 umfasst im wesentlichen eine Röntgenquelle 30 und einen Empfänger 29, welcher auf magneto-optischer Basis ein Röntgenbild 35 senkrecht zur Bildnormalen 31;32 erstellt. Die Röntgenbilder 35 des Patienten 33 werden im wesentlich anterior-posterior und lateral-medial oder umgekehrt aufgenommen, so dass die Bildnormale 31;32 ungefähr senkrecht oder waagrecht verläuft, je nach Position des Röntgengerätes 28. Das erfindungsgemässe Verfahren umfasst folgende Schritte:
a) Erfassen und Speichern der Richtung des Schwerkraftvektors 19 innerhalb eines dreidimensionalen Koordinatensystems 5;24;26 mittels der Positionserfassungsvorrichtung 12 und des Computers 8. Ein raumfestes Koordinatensystem 24 kann durch Ausmessen von mindestens drei nicht-kollinear, fest im Raum angeordneten Markern 21 mittels der positionserfassungsvorrichtung 12 ermittelt werden, während die Ermittlung des schwerkraftfesten dreidimensionalen Koordinatensystems 5, welches ebenfalls raumfest ist, durch Ausmessen der Horizontalachsen 4;7 der Inklinometer 3;6 und deren Schnittpunkt oder durch Ausmessen von mindestens drei nicht-kollinear am Körper 20 angeordneten Markern 21 erfolgen kann;
b) Ermitteln der Position und Orientierung der Röntgenquelle 30 und/oder des Empfängers 29 innerhalb desselben Koordinatensystems 5;24;26 mittels der Positionserfassungsvorrichtung 12 und des Computers 8 durch Ausmessen der Position der an der Röntgenquelle 30 und/oder am Empfänger 29 befestigten Marker 21;
c) Ermitteln der aufgrund von schwerkraftbedingten mechanischen Deformationen des Röntgengerätes 28 auftretenden Verzerrungen des Röntgenbildes mittels des Computers 8;
d) Korrektur des Röntgenbildes bezüglich der unter Schritt c) ermittelten Verzerrungen mittels des Computers 8;
e) Ermitteln der Richtung der Bildnormalen 31;32 innerhalb desselben Koordinatensystems 5;24;26 aus der unter Schritt b) ermittelten Position und Orientierung des Empfängers 29 mittels des Computers 8;
f) Ermitteln der aufgrund der Abweichung der Bildnormalen 31;32 von der Richtung des lokalen Erdmagnetfeldes auftretenden Defekte des auf magneto-optischer Basis vom Empfänger 29 erzeugten Röntgenbildes mittels des Computers 8;
g) Korrektur des Röntgenbildes bezüglich der unter Schritt f) ermittelten Defekte mittels des Computers 8;
h) Ermitteln der Defekte auf das magneto-optische Röntgenbild 35, welche aufgrund von optischen Deformationen im Empfänger 29 verursacht werden und insbesondere durch die vertikale oder horizontale Ausrichtung des Röntgengerätes 28 mitbeeinflusst werden;
i) Korrektur des Röntgenbildes 35 bezüglich der unter Schritt h) ermittelten Defekte mittels des Computers 8.

## Patentansprüche

1. Vorrichtung zur Erfassung der Position und Orientierung eines mit mindestens drei nicht-kollinear angeordneten Markern (21) versehenen Körpers (36) innerhalb mindestens eines dreidimensionalen Koordinatensystems (5;24;26) mit
A) einem Positionserfassungssensor (1) zur Ortung der Marker (21) innerhalb desselben Koordinatensystems (5;24;26) oder eines weiteren dreidimensionalen Koordinatensystems (5;24;26); und
B) einem Computer (8) zur Ermittlung der Position und Orientierung des mit mindestens drei nicht-kollinear angeordneten Markern (21) versehenen Körpers (36) innerhalb mindestens eines Koordinatensystems (5;24;26) aus den durch den Positionserfassungssensor (1) bestimmten Orten von den Markern (21),
**dadurch gekennzeichnet, dass**
C) die Vorrichtung Hilfsmittel (25) zur Ermittlung der Richtung des Schwerkraftvektors (19) innerhalb eines Koordinatensystems (5;24;26) umfasst.

2. Vorrichtung nach Anspruch 1, **dadurch gekennzeichnet, dass** die Hilfsmittel (25) mindestens ein Inklinometer (4) umfassen.

3. Vorrichtung nach Anspruch 2, **dadurch gekennzeichnet, dass** die Hilfmittel (25) zwei Inklinometer (3;6) umfassen, welche so am Positionserfassungssensor (1) befestigt sind, dass ihre Horizontalachsen (4;7) senkrecht zueinander verlaufen.

4. Vorrichtung nach Anspruch 2 oder 3, **dadurch gekennzeichnet, dass** das oder die Inklinometer (3;6) nach dem Gasblasen/Flüssigkeitsprinzip analog zu einer Wasserwaage arbeiten und Abweichungen der Horizontalachsen (4;7) gegenüber dem Schwerkraftvektor (19) elektronisch erfassen.

5. Vorrichtung nach Anspruch 2 oder 3, **dadurch gekennzeichnet, dass** das oder die Inklinometer (3;6) nach dem Kreiselprinzip mit raumfesten Drehimpulsvektor analog zu einem Kreiselkompass arbeiten und Abweichungen der kalibrierten Horizontalachsen (4;7) gegenüber dem Schwerkraftvektor (19) elektronisch erfassen.

6. Vorrichtung nach Anspruch 2 oder 3, **dadurch gekennzeichnet, dass** das oder die Inklinometer (3;6) nach dem Trägheitsprinzip analog zur Trägheitspositionsbestimmung arbeiten und Abweichungen der kalibrierten Horizontalachsen (4;7) gegenüber dem Schwerkraftvektor (19) elektronisch erfassen.

7. Vorrichtung nach Anspruch 1, **dadurch gekennzeichnet, dass** die Hilfsmittel (25) einen im Raume aufgehängten, sich im lokalen Schwerefeld ausrichtenden Körper (20) mit mindestens zwei im Abstand A angeordneten Markern (21) umfassen.

8. Vorrichtung nach Anspruch 7, **dadurch gekennzeichnet, dass** die Aufhängung (27) des Körpers (20) mittels eines dünnen Fadens oder Drahtes (22) realisiert ist.

9. Vorrichtung nach Anspruch 7, **dadurch gekennzeichnet, dass** die Aufhängung (27) des Körpers (20) mittels einer Kette realisiert ist.

10. Vorrichtung nach Anspruch 7, **dadurch gekennzeichnet, dass** die Aufhängung (27) des Körpers (20) eine kardanische Aufhängung ist.

11. Vorrichtung nach Anspruch 7, **dadurch gekennzeichnet, dass** die Aufhängung (27) des Körpers (20) mittels eines Kugelgelenkes realisiert ist.

12. Vorrichtung nach Anspruch 7, **dadurch gekennzeichnet, dass** die Aufhängung (27) des Körpers (20) im Raum durch Einlagerung des Körpers (20) in ein Elastomer gestaltet ist.

13. Vorrichtung nach Anspruch 12, **dadurch gekennzeichnet, dass** das Elastomer ein Silikonkautschuk ist.

14. Vorrichtung nach Anspruch 13, **dadurch gekennzeichnet, dass** der Körper (20) in einen geschäumten Silikonkautschuk eingelagert ist.

15. Vorrichtung nach einem der Ansprüche 7 bis 14, **dadurch gekennzeichnet, dass** die Ausrichtung des Körpers (20) im Schwerefeld gedämpft ist.

16. Vorrichtung nach Anspruch 15, **dadurch gekennzeichnet, dass** die Ausrichtung des Körpers (20) im Schwerefeld durch ein den Körper (20) umgebendes Flüssigkeitsbad gedämpft ist.

17. Vorrichtung nach Anspruch 15, **dadurch gekennzeichnet, dass** die Ausrichtung des Körpers (20) im Schwerefeld durch Federn gedämpft ist.

18. Vorrichtung nach Anspruch 15, **dadurch gekennzeichnet, dass** die Ausrichtung des Körpers (20) im Schwerefeld elektromagnetisch gedämpft ist.

19. Vorrichtung nach Anspruch 15, **dadurch gekennzeichnet, dass** die Ausrichtung des Körpers (20) im Schwerefeld durch Reibung mechanisch gedämpft ist.

20. Vorrichtung nach Anspruch 15, **dadurch gekennzeichnet, dass** die Ausrichtung des Körpers (20) im Schwerefeld durch Luftoder Gasdruckstossdämpfer gedämpft ist.

21. Vorrichtung nach einem der Ansprüche 1 bis 20, **dadurch gekennzeichnet, dass** die Marker (21) Infrarot Light Emitting Dioden (IRED) sind.

22. Vorrichtung nach einem der Ansprüche 1 bis 21, **dadurch gekennzeichnet, dass** der Positionserfassungssensor (1) optoelektronisch arbeitet und mindestens zwei Kameras (2) umfasst, deren optische Achsen (14;15;16) sich in einem Punkt (17) schneiden.

23. Vorrichtung nach einem der Ansprüche 1 bis 22, **dadurch gekennzeichnet, dass** zum Betreiben der Positionserfassungsvorrichtung (12) der Computer (8) mit einer Interface-Karte oder Schnittstelle (11) ausgestattet ist und die Vorrichtung eine System-Kontrolleinheit (9) umfasst.

24. Verfahren zur Korrektur von Röntgenbildern (35) bezüglich Defekten, welche durch schwerkraftbedingte mechanische Deformationen eines eine Röntgenquelle (30) und einen Empfänger (29) umfassenden Röntgengerätes (28) auftreten, mittels der Vorrichtung gemäss einem der Ansprüche 1 bis 23, **dadurch gekennzeichnet, dass** das Verfahren folgende Schritte umfasst:
a) Erfassen der Richtung des Schwerkraftvektors (19) innerhalb eines dreidimensionalen Koordinatensystems (5;24;26) mittels der Positionserfassungsvorrichtung (12) und des Computers (8);
b) Ermitteln der Position und Orientierung des Röntgengerätes (28) innerhalb desselben Koordinatensystems (5;24;26) mittels der Positionserfassungsvorrichtung (12) und des Computers (8) durch Ausmessen der Position von mindestens drei nicht-kollinear angeordneten am Röntgengerät (28) befestigten Markern (21);
c) Ermitteln der Defekte des Röntgenbildes (35) aus der unter Schritt b) ermittelten Position und Orientierung des Röntgengerätes (28) mittels des Computers (8); und
d) Korrektur des digitalen Röntgenbildes bezüglich der unter Schritt c) ermittelten Defekte mittels des Computers (8).

25. Verfahren nach Anspruch 24, **dadurch gekennzeichnet, dass** es zusätzlich das Digitalisieren des Röntgenbildes (35) und Erfassen des digitalisierten Röntgenbildes (35) im Computer (8) umfasst.

26. Verfahren nach Anspruch 24 oder 25, **dadurch gekennzeichnet, dass** vom Empfänger (29) ein magneto-optisches Röntgenbild (35) erzeugt wird.

27. Verfahren nach Anspruch 26, **dadurch gekennzeichnet, dass** es zusätzlich die folgenden Schritte umfasst:
f) Ermitteln der Richtung der senkrecht auf dem Röntgenbild (35) stehenden Bildnormalen (31;32) innerhalb desselben Koordinatensystems (5;24;26) aus der unter Schritt b) ermittelten Position und Orientierung des Röntgengerätes (28);
g) Ermitteln der erdmagnetfeldbedingten Defekte des magneto-optischen Röntgenbildes (35) aus der Position und Orientierung des Röntgengerätes (28) relativ zum Schwerkraftvektor (19) mittels des Computers (8); und
h) Korrektur des digitalisierten Röntgenbild (35) bezüglich dieser Defekte mittels des Computers (8).

28. Verfahren nach einem der Ansprüche 24 bis 27, **dadurch gekennzeichnet dass** die Position und Orientierung der Röntgenquelle (30) innerhalb des Koordinatensystems (5;24;26) mittels der Positionserfassungsvorrichtung (12) und des Computers (8) durch Ausmessen der Position von mindestens drei an der Röntgenquelle (30) befestigten Markern (21) ermittelt wird.

29. Verfahren nach einem der Ansprüche 24 bis 28, **dadurch gekennzeichnet dass** die Position und Orientierung des Empfängers (29) innerhalb des Koordinatensystems (5;24;26) mittels der Positionserfassungsvorrichtung (12) und des Computers (8) durch Ausmessen der Position von mindestens drei am Empfänger (29) befestigten Markern (21) ermittelt wird.

30. Verfahren nach Anspruch 29, **dadurch gekennzeichnet, dass** zusätzlich Defekte des Röntgenbildes (35), welche durch optische Deformationen des magneto-optischen Röntgenbildes (35) im Empfänger (29) verursacht werden, aus der Position und Orientierung des Empfängers (29) ermittelt werden und das digitalisierte Röntgenbild (35) entsprechend korrigiert wird.

## Claims

1. A device for detecting the position and orientation of a body (36) provided with at least three non-collinear markers (21) within at least one three-dimensional coordinate system (5;24;26) including
A) a position sensor (1) for locating the markers (21) within said coordinate system (5;24;26) or within another three-dimensional coordinate system (5;24;26); and
B) a computer (8) for determining the position and orientation of the body (36) provided with at least three non-collinear markers (21) within at least one coordinate system (5;24;26) from the places of the markers (21) as detected by the position sensor (1),
**characterized in that**
C) the device comprises auxiliary means (25) for determining the direction of the gravity vector (19) within a coordinate system (5;24;26).

2. A device as claimed in claim 1, **characterised in that** the auxiliary means (25) comprise at least one inclinometer (4).

3. A device as claimed in claim 2, **characterised in that** the auxiliary means (25) comprise two inclinometers (3;6) which are fastened to the position sensor (1) in such a way that their horizontal axes (4;7) extend perpendicularly to each other.

4. A device as claimed in claim 2 or 3, **characterised in that** the inclinometer(s) (3;6) is (are) based on the principle of gas bubble and liquid, by analogy with a spirit level, and electronically detect(s) deviations of the horizontal axes (4;7) relative to the gravity vector (19).

5. A device as claimed in claim 2 or 3, **characterised in that** the inclinometer(s) (3;6) is (are) based on the principle of gyration with a space-based vector of angular momentum, by analogy with a gyrocompass, and electronically detect(s) deviations of the calibrated horizontal axes (4;7) relative to the gravity vector (19).

6. A device as claimed in claim 2 or 3, **characterised in that** the inclinometer(s) (3;6) is (are) based on the principle of inertia, by analogy with inertial position detection, and electronically detect(s) deviations of the calibrated horizontal axes (4;7) relative to the gravity vector (19).

7. A device as claimed in claim 1, **characterised in that** the auxiliary means (25) comprise a body (20) suspended in space and aligning with the local gravitational field, said body being equipped with at least two markers (21) arranged at a distance A from each other.

8. A device as claimed in claim 7, **characterised in that** the suspension (27) of the body (20) is realised by means of a thin thread or wire (22).

9. A device as claimed in claim 7, **characterised in that** the suspension (27) of the body (20) is realised by means of a chain.

10. A device as claimed in claim 7, **characterised in that** the suspension (27) of the body (20) is a cardanic suspension.

11. A device as claimed in claim 7, **characterised in that** the suspension (27) of the body (20) is realised by means of a ball-and-socket bearing.

12. A device as claimed in claim 7, **characterised in that** the suspension (27) of the body (20) in space is realised by means of an embedding of the body (20) in an elastomer.

13. A device as claimed in claim 12, **characterised in that** the elastomer is a silicone rubber.

14. A device as claimed in claim 13, **characterised in that** the body (20) is embedded in a foamed silicone rubber.

15. A device as claimed in any of the claims 7 to 14, **characterised in that** the alignment of the body (20) with the gravitational field is shock-absorbed.

16. A device as claimed in claim 15, **characterised in that** the alignment of the body (20) with the gravitational field is shock-absorbed by means of an immersion of said body (20) into a liquid.

17. A device as claimed in claim 15, **characterised in that** the alignment of the body (20) with the gravitational field is shock-absorbed by means of springs.

18. A device as claimed in claim 15, **characterised in that** the alignment of the body (20) with the gravitational field is shock-absorbed by electromagnetic means.

19. A device as claimed in claim 15, **characterised in that** the alignment of the body (20) with the gravitational field is mechanically shock-absorbed by means of friction.

20. A device as claimed in claim 15, **characterised in that** the alignment of the body (20) with the gravitational field is shock-absorbed by means of shock absorbers based on air pressure or gas pressure.

21. A device as claimed in any of the claims 1 to 20, **characterised in that** the markers (21) are infrared light-emitting diodes (IREDs).

22. A device as claimed in any of the claims 1 to 21, **characterised in that** the position sensor (1) is an optoelectronic sensor and comprises at least two cameras (2) the optical axes (14;15;16) of which intersect each other at a point (17).

23. A device as claimed in any of the claims 1 to 22, **characterised in that** for operating the position detector (12) the computer (8) is equipped with an interface card (11) and the device comprises a System Control Unit (9).

24. A method for correcting X-ray photographs (35) with respect to shortcomings arising from gravity-induced, mechanical deformations of an X-ray apparatus (28) comprising an X-ray source (30) and a receiver (29) by means of the device according to any of the claims 1 to 23, **characterised in that** said method comprises the following steps:
a) detecting the direction of the gravity vector (19) within a three-dimensional coordinate system (5;24;26) by means of the position detector (12) and the computer (8).
b) determining the position and orientation of the X-ray apparatus (28) within said coordinate system (5;24;26) by means of the position detector (12) and the computer (8) by measuring the position of the at least three markers (21) fixed on the X-ray apparatus (28) in a non-collinear arrangement;
c) determining the shortcomings of the X-ray photograph (35) from the position and orientation of the X-ray apparatus (28) as determined in step b) by means of the computer (8); and
d) correcting the digital X-ray photograph with respect to the shortcomings determined in step c) by means of the computer (8).

25. A method as claimed in claim 24, **characterised in that** it further comprises the digitizing of the X-ray photograph (35) and the storing the digitized X-ray photograph (35) in the computer (8).

26. A method as claimed in claim 24 or 25, **characterised in that** a magneto-optical X-ray photograph (35) is generated by the receiver (29).

27. A method as claimed in claim 26, **characterised in that** it further comprises the following steps:
e) determining the direction of the normal (31;32) extending perpendicularly to the X-ray photograph (35) within said coordinate system (5;24;26) from the position and orientation of the X-ray apparatus (28) as determined in step b);
f) determining the shortcomings of the X-ray photograph (35) caused by the Earth's magnetic field from the position and orientation of the X-ray apparatus (28) relative to the gravity vector (19) by means of the computer (8); and
g) correction of the digitized X-ray photograph (35) with respect to said shortcomings by means of the computer (8).

28. A method as claimed in any of the claims 24 to 27, **characterised in that** the position and orientation of the X-ray source (30) within the coordinate system (5;24;26) is determined by means of the position detector (12) and the computer (8) by measuring the position of at least three markers (21) fixed on the X-ray source (30).

29. A method as claimed in any of the claims 24 to 28, **characterised in that** the position and orientation of the receiver (29) within the coordinate system (5;24;26) is determined by means of the position detector (12) and the computer (8) by measuring the position of at least three markers (21) fixed on the receiver (29).

30. A method as claimed in claim 29, **characterised in that**, in addition, shortcomings of the X-ray photograph (35) which are caused by optical deformations of the magneto-optical X-ray photograph (35) occurring in the receiver (29) are determined from the position and orientation of the receiver (29) and that the digitized X-ray photograph (35) is accordingly corrected.

## Revendications

1. Dispositif servant à détecter la position et l'orientation d'un corps (36) pourvu d'au moins trois repères (21) disposés de manière non colinéaire dans au moins un système de coordonnées à trois dimensions (5, 24, 26) comprenant
A) un capteur (1) de détection de position servant à localiser les repères dans le même système de coordonnées (5, 24, 26) ou dans un autre système de coordonnées à trois dimensions (5, 24, 26), et
B) un ordinateur (8) servant à déterminer la position et l'orientation du corps (36) pourvu d'au moins trois repères (21) disposés de manière non colinéaire dans au moins un système de coordonnées (5, 24, 26) en partant des emplacements des repères (21) déterminés par le capteur (1) de détection de position,
**caractérisé en ce que**
C) le dispositif comprend des moyens auxiliaires (25) pour déterminer la direction du vecteur de gravité (19) dans un système de coordonnées (5, 24, 26).

2. Dispositif selon la revendication 1, **caractérisé en ce que** les moyens auxiliaires (25) comprennent au moins un inclinomètre (3).

3. Dispositif selon la revendication 2, **caractérisé en ce que** les moyens auxiliaires (25) comprennent deux inclinomètres (3, 6) qui sont fixés sur le capteur (1) de détection de position de telle manière que leurs axes horizontaux (4, 7) s'étendent perpendiculairement l'un à l'autre.

4. Dispositif selon la revendication 2 ou 3, **caractérisé en ce que** l'inclinomètre ou les inclinomètres (3, 6) fonctionnent selon le principe de la bulle de gaz et du liquide de manière analogue à un niveau à bulle d'air et détectent électroniquement les écarts des axes horizontaux (4, 7) par rapport au vecteur de gravité (19).

5. Dispositif selon la revendication 2 ou 3, **caractérisé en ce que** l'inclinomètre ou les inclinomètres (3, 6) fonctionnent selon le principe du gyroscope avec un vecteur d'impulsion de rotation fixé dans l'espace de manière analogue à un compas gyroscopique et déterminent électroniquement les écarts des axes horizontaux (4, 7) calibrés par rapport au vecteur de gravité (19).

6. Dispositif selon la revendication 2 ou 3, **caractérisé en ce que** l'inclinomètre ou les inclinomètres (3, 6) fonctionnent selon le principe d'inertie de manière analogue à la détermination de la position par inertie et déterminent électroniquement les écarts des axes horizontaux (4, 7) calibrés par rapport au vecteur de gravité (19).

7. Dispositif selon la revendication 1, **caractérisé en ce que** les moyens auxiliaires (25) comprennent un corps (20) suspendu dans l'espace, s'orientant dans le champ de gravité local, qui comporte au moins deux repères (21) disposés à une distance A.

8. Dispositif selon la revendication 7, **caractérisé en ce que** la suspension (27) du corps (20) est réalisée au moyen d'un fin fil ou câble (22).

9. Dispositif selon la revendication 7, **caractérisé en ce que** la suspension (27) du corps (20) est réalisée au moyen d'une chaîne.

10. Dispositif selon la revendication 7, **caractérisé en ce que** la suspension (27) du corps (20) est une suspension à la Cardan.

11. Dispositif selon la revendication 7, **caractérisé en ce que** la suspension (27) du corps (20) est réalisée au moyen d'une rotule.

12. Dispositif selon la revendication 7, **caractérisé en ce que** la suspension (27) du corps (20) dans l'espace est réalisée par inclusion du corps (20) dans un élastomère.

13. Dispositif selon la revendication 12, **caractérisé en ce que** l'élastomère est un caoutchouc silicone.

14. Dispositif selon la revendication 13, **caractérisé en ce que** le corps (20) est inclus dans une mousse de caoutchouc silicone.

15. Dispositif selon une des revendications 7 à 14, **caractérisé en ce que** l'orientation du corps (20) est amortie dans le champ de gravité.

16. Dispositif selon la revendication 15, **caractérisé en ce que** l'orientation du corps (20) est amortie dans le champ de gravité par un bain de liquide entourant le corps (20).

17. Dispositif selon la revendication 15, **caractérisé en ce que** l'orientation du corps (20) est amortie dans le champ de gravité par des ressorts.

18. Dispositif selon la revendication 15, **caractérisé en ce que** l'orientation du corps (20) est amortie électromagnétiquement dans le champ de gravité.

19. Dispositif selon la revendication 15, **caractérisé en ce que** l'orientation du corps (20) est amortie mécaniquement dans le champ de gravité par friction.

20. Dispositif selon la revendication 15, **caractérisé en ce que** l'orientation du corps (20) est amortie dans le champ de gravité par des amortisseurs à air comprimé ou à gaz comprimé.

21. Dispositif selon une des revendications 1 à 20, **caractérisé en ce que** les repères (21) sont des diodes électroluminescentes infrarouges (IRED).

22. Dispositif selon une des revendications 1 à 21, **caractérisé en ce que** le capteur (1) de détection de position fonctionne de manière opto-électronique et comprend au moins deux caméras (2) dont les axes optiques (14, 15, 16) se coupent en un point (17).

23. Dispositif selon une des revendications 1 à 22, **caractérisé en ce que** pour faire fonctionner le dispositif de détection de position (12), l'ordinateur est équipé d'une carte d'interface ou d'une interface (11) et le dispositif comprend une unité de contrôle (9) du système.

24. Procédé servant à corriger des radiographies (35) en ce qui concerne les défauts qui surviennent à cause des déformations mécaniques dues à la gravité d'un appareil de radiographie (28) comprenant une source de rayons X (30) et un récepteur (29), au moyen du dispositif conforme à une des revendications 1 à 23, **caractérisé en ce que** le procédé comprend les étapes suivantes :
a) détecter la direction du vecteur de gravité (19) dans un système de coordonnées à trois dimensions (5, 24, 26) au moyen d'un dispositif de détection de position (12) et de l'ordinateur (8).
b) déterminer la position et l'orientation de l'appareil de radiographie (28) dans le même système de coordonnées (5, 24, 26) au moyen du dispositif de détection de position (12) et de l'ordinateur (8) en mesurant la position d'au moins trois repères (21) fixés de manière non colinéaire sur l'appareil de radiographie (28),
c) déterminer les défauts de la radiographie (35) au moyen de l'ordinateur (8) en partant de la position et de l'orientation de l'appareil de radiographie (28) déterminées à l'étape b), et
d) corriger la radiographie numérique au moyen de l'ordinateur (8) en ce qui concerne les défauts déterminés à l'étape c).

25. Procédé selon la revendication 24, **caractérisé en ce qu'**il comprend en outre la numérisation de la radiographie (35) et l'enregistrement de la radiographie (35) numérisée dans l'ordinateur (8).

26. Procédé selon la revendication 24 ou 25, **caractérisé en ce qu'**une radiographie (35) magnéto-optique est produite par le récepteur (29).

27. Procédé selon la revendication 26, **caractérisé en ce qu'**il comprend en outre les étapes suivantes :
f) déterminer la direction des normales (31, 32) de l'image perpendiculaires à la radiographie (35) dans le même système de coordonnées (5, 24, 26) en partant de la position et de l'orientation de l'appareil de radiographie (28) déterminées à l'étape b),
g) déterminer au moyen de l'ordinateur (8) les défauts de la radiographie (35) magnéto-optique dus au champ magnétique terrestre en partant de la position et de l'orientation de l'appareil de radiographie (28) par rapport au vecteur de gravité (19), et
h) corriger au moyen de l'ordinateur (8) la radiographie (35) numérisée en ce qui concerne ces défauts.

28. Procédé selon une des revendications 24 à 27, **caractérisé en ce que** la position et l'orientation de la source de rayons X (30) sont déterminées dans le système de coordonnées (5, 24, 26) au moyen du dispositif de détection de position (12) et de l'ordinateur (8) en mesurant la position d'au moins trois repères (21) fixés sur la source de rayons X (30).

29. Procédé selon une des revendications 24 à 28, **caractérisé en ce que** la position et l'orientation du récepteur (29) sont déterminées dans le système de coordonnées (5, 24, 26) au moyen du dispositif de détection de position (12) et de l'ordinateur (8) en mesurant la position d'au moins trois repères (21) fixés sur le récepteur (29).

30. Procédé selon la revendication 29, **caractérisé en ce qu'**en outre, les défauts de la radiographie (35) qui sont provoqués par des déformations optiques de la radiographie (35) magnéto-optique sont, en outre, déterminés à partir de la position et de l'orientation du récepteur (29) et la radiographie (35) numérisée est corrigée en conséquence.
